# EUROPÄISCHE PATENTSCHRIFT

(11) **EP 2 712 653 B1**
(45) Veröffentlichungstag und Bekanntmachung des Hinweises auf die Patenterteilung: **27.12.2017**
(21) Anmeldenummer: 13176721.2
(22) Anmeldetag: 16.07.2013
(51) Int. Cl.: A61M 39/26, F16K 15/02, A61M 39/24

(54) **Fluidanschluss**
Fluid coupling
Raccord de fluide

(30) Priorität: 27.09.2012 DE 102012109192
(43) Veröffentlichungstag der Anmeldung: 02.04.2014
(73) Patentinhaber: B. Braun Avitum AG, 34212 Melsungen (DE)
(72) Erfinder: Orszullok, Willy, 58809 Neuenrade (DE)
(74) Vertreter: Winter, Brandl, Fürniss, Hübner, Röss, Kaiser, Polte - Partnerschaft mbB

(56) Entgegenhaltungen:
- EP-A1- 1 371 579
- EP-A1- 1 839 699
- WO-A1-98/11931
- FR-A1- 2 777 229
- US-A1- 2010 292 674
- US-B1- 7 160 272

## Beschreibung

Die vorliegende Erfindung betrifft einen Fluidanschluss nach dem Oberbegriff des Anspruchs 1.

Aus den verschiedensten Bereichen der Fluidtechnik sind Ventile mit federbelastetem Dichtungsabschnitt, insbesondere Dichtkegel, bekannt. Im Bereich der Medizintechnik ist zum Beispiel in der WO 2008/04877 ein über einen Luer-Mechanismus betätigbares Ventil offenbart, durch das eine bidirektionale Strömung eines das Ventil durchströmenden, medizinischen Mediums reguliert wird.

Aus der EP 0 261 317 B1, ist ferner eine Rückschlagventilvorrichtung bekannt. Weiterhin zeigt die WO 98/11931 A1 einen Fluidanschluss mit den Merkmalen des Oberbegriffs des Anspruchs 1. Allen bekannten Anwendungen gemeinsam ist, dass zum Erzeugen der erforderlichen Dichtelement-Vorspannung gegenüber einem korrespondierenden Ventilsitz ein separates Federelement sowie ein separates Abstützelement oder Federsitz für das Federelement erforderlich sind.

Es ist daher eine Aufgabe der vorliegenden Erfindung, die Anzahl der Bauteile eines solchen Ventils zu verringern und gleichzeitig dessen Funktionssicherheit zu erhöhen, wobei durch letzteres insbesondere die pharmazeutisch-hygienischen Eigenschaften des Ventils insofern verbessert werden, dass zum Beispiel eine schnelle Verhinderung einer Rückströmung des medizinischen Mediums gewährleistet ist.

Diese Aufgabe wird durch die Lehre des Anspruchs 1 gelöst.

Auch ohne jeweils explizit darauf hinzuweisen, sind alle im Folgenden genannten, vorzugsweise diskutierten Merkmale entweder 'erfindungsgemäß', bilden also den Anspruch 1, oder nicht erfindungsgemäß in diesem Sinne, jedoch ,zur vorliegenden Erfindung gehörend', definieren also vorteilhafte Weiterbildungen (Unteransprüche) der vorliegenden Erfindung oder Modifikationen davon. Ferner sind alle Kombinationen dieser Merkmale Teil der Erfindung, sofern sie funktional vereinbar sind und sich nicht gegenseitig ausschließen.

Erfindungsgemäß (Anspruch 1) hat ein Fluidanschluss beispielsweise eines medizinischen Behälters einen Anschluss-Stutzen und ein darin angeordnetes selbstschließendes Ventil beziehungsweise Sitzventil / Rückschlagventil, wobei der Anschluss-Stutzen einen Strömungskanal ausbildet, der von einem, vorzugsweise medizinischen Fluid in einer Strömungsrichtung (entgegengesetzt zur Öffnungsrichtung des Rückschlagventils) durchströmbar ist. In dem Anschluss-Stutzen ist eine Ventilsitzeinrichtung ausgebildet, wobei das Ventil einen mit dem Anschluss-Stutzen lösbar verbundenen Halteabschnitt beziehungsweise ein Federsitzelement aufweist. Das Ventil ist dadurch gekennzeichnet, dass es einen entlang einer gemeinsamen Längsachse des Anschluss-Stutzens und des Ventils zwischen einer vollständig geöffneten Position und einer vollständig geschlossenen Position bewegbaren Dichtungsabschnitt / Ventilkörper aufweist, der in der vollständig geschlossenen Position mit der Ventilsitzeinrichtung dichtend in Eingriff ist und der Halteabschnitt / Federsitz einteilig und federelastisch (über eine Feder) mit dem Dichtungsabschnitt verbunden ist.

In anderen Worten ausgedrückt bildet der Anschluss-Stutzen eine Art (Rückschlag-) Ventilpatrone mit einer im Anschluss-Stutzen ausgeformten Ventilsitzeinrichtung und einer in den Anschluss-Stutzen eingesetzten Schließeinheit bestehend aus dem mit der Ventilsitzeinrichtung zusammenwirkenden Ventilkörper / Dichtungsabschnitt, der in Schließrichtung wirkenden Feder und dem Federsitzelement / Halteabschnitt, das mit dem Anschluss-Stutzen fest verbunden/verbindbar ist. Erfindungsgemäß sind der Ventilkörper, die Feder und das Federsitzelement / Halteelement stoffeinstückig ausgeformt / ausgebildet.

Das "Fluid" ist eine Flüssigkeit / Granulat oder ein Gas. Der "Fluidanschluss" ist ein Mittel, das, eingebaut / ausgeformt in eine Wand eines Behälters oder in eine Leitung, vorzugsweise einen Schlauch, dazu geeignet ist, das Strömen in den oder aus dem Behälter bzw. das Durchströmen durch die Leitung des Fluids zu regulieren. Das "Ventil" ist eine Baueinheit des Fluidanschlusses, das wenigstens einen Abschnitt, den "Dichtungsabschnitt" (Ventilkörper), aufweist, der relativ zu dem Anschluss-Stutzen durch Fluiddruck und seine federelastische Verbindung mit dem Halteabschnitt / Federsitz in einer Schließrichtung und durch Betätigung von außen in einer zu der Schließrichtung entgegengesetzten Schließrichtung bewegbar ist, wobei diese Bewegung von der Ventilsitzeinrichtung weg bzw. auf diese zu und schließlich in Anlage an sie gelangend erfolgt, und zwar relativ zu dem Anschluss-Stutzen und dem mit diesem verbundenen Halteabschnitt. Die Bewegungsachse dieser Bewegung ist gleich der Längsachse des Anschluss-Stutzens und des darin angeordneten Ventils. Vorzugsweise begrenzen der Halteabschnitt und der Dichtungsabschnitt zusammen mit dem Anlass-Stutzen einen (Aufnahme-) Raum, in den der Dichtungsabschnitt beim Öffnen des Ventils eindringt, um so von dem Fluid umströmt zu werden, so dass in strömendem Zustand des Fluids der Halteabschnitt (vorzugsweise in Form einer Ringplatte oder Hülse) von dem Fluid , "durch"strömt wird, während der Dichtungsabschnitt (vorzugsweise in Form einer Schließkappe oder Ventiltellers) von dem Fluid "um"strömt wird.

Das Fluid tritt bestimmungsgemäß auf der Seite des Halteabschnitts in den Fluidanschluss ein, so dass die Strömungsrichtung von dem Halteabschnitt in Richtung hin zum Dichtungsabschnitt definiert ist. Ferner ist das Fluid vorzugsweise ein medizinisches Fluid, vorzugsweise eine Infusionslösung oder eine Blutkonserve oder dergleichen, das steril in dem Behälter aufgenommen ist. Die federelastische Verbindung zwischen dem Dichtungsabschnitt und dem Halteabschnitt erzeugt vorzugsweise eine diese beiden Abschnitte beabstandende Kraft in Schließrichtung des Ventils, die die ebenfalls in diese Richtung wirkende Schließkraft des strömenden / sich anstauenden Fluids unterstützt.

Gemäß einer vorteilhaften Weiterbildung der vorliegenden Erfindung (Anspruch 2) wird die federelastische Verbindung durch wenigstens eine Feder, vorzugsweise wenigstens eine Blattfeder, vorzugsweise wenigstens eine geschwungene Blattfeder oder eine V-förmige Blattfeder oder eine Z-förmige Blattfeder oder eine ellipsenförmige Blattfeder, oder vorzugsweise durch eine elastische schlauchartige Gitter- / Fachwerkstruktur erzeugt.

Vorzugsweise erstreckt sich die wenigstens eine Feder im Wesentlichen durch den Raum, der von dem Halteabschnitt, dem Dichtungsabschnitt und dem Anschluss-Stutzen begrenzt ist.

Gemäß einer vorteilhaften Weiterbildung der vorliegenden Erfindung (Anspruch 3) wird die federelastische Verbindung durch wenigstens eine Feder, vorzugsweise eine schräg gestellte Biegestabfeder erzeugt.

Gemäß einer vorteilhaften Weiterbildung der vorliegenden Erfindung (Anspruch 4) ist der Halteabschnitt im Wesentlichen in Form eines Rings / einer Ringscheibe und das Dichtelement im Wesentlichen in Form einer Scheibe / Ventilteller ausgebildet, wobei die federelastische Wirkung durch eine Mehrzahl von Federn erzeugt wird, die im Wesentlichen äquidistant entlang des Halteabschnitts bzw. eines Umfangsabschnitts des Dichtungsabschnitts geschaltet sind.

Vorzugsweise weist der Dichtungsabschnitt/Ventilkörper einen ringförmigen Umfangabschnitt auf, der dem ringförmigen Halteabschnitt in Richtung der Längsachse gegenüberliegt, so dass ein Mittelpunkt des Halteabschnitt-Rings eine feste Position auf der Längsachse einnimmt und sich ein Mittelpunkt des Dichtungsabschnitt-Umfangsrings beim Öffnen des Ventils entgegen der Strömungsrichtung und beim Schließen des Ventils in der Strömungsrichtung auf der Längsachse bewegt. Das heißt, durch eine äquidistante Anordnung von mehreren Federn wird ein Verkippen des Ventilkörpers und damit ein Verkannten und Blockieren des Ventils im Wesentlichen vermieden. Bei einer geeigneten Federanordnung der Mehrzahl von Federn, vorzugsweise bei den genannten schräg gestellten Biegefedern, bewegt sich der Ventilkörper nicht nur entlang der Längsachse, sondern führt zudem um diese eine Drehbewegung aus, die einen stabiliserenden, d. h. einer Kippneigung des Ventilkörpers entgegenwirkenden Effekt hat.

Gemäß einer vorteilhaften Weiterbildung der vorliegenden Erfindung (Anspruch 5) ist die wenigstens eine Feder durch Urformen, vorzugsweise Spritzgießen, gebildet und mit dem Halteabschnitt und dem Dichtungsabschnitt stoffeinstückig verbunden.

Vorzugsweise (i) weist der Halteabschnitt (Federsitz) und der Dichtungsabschnitt (Ventilkörper) jeweils einen, vorzugsweise Form gebenden und stützenden Kern auf, der umspritzt ist, um so eine Ummantelung zu bilden, und (ii) bildet die Ummantelung mit der bei diesem Prozess ebenfalls und einteilig mit dieser gebildeten wenigstens einen Feder eine Einheit aus Halteabschnitt, Feder und Dichtungsabschnitt. Vorzugsweise kann auch der Halteabschnitt, die Feder und der Dichtungsabschnitt ein einziges (kernloses), durch Spritzgießen oder ein anderes geeignetes Urformverfahren hergestelltes Bauteil bilden. Ferner können erfindungsgemäß der Halteabschnitt, der Dichtungsabschnitt und die wenigstens eine Feder aus verschiedenen, vorzugsweise ein, zwei, oder drei, Materialien gebildet sein. Vorzugsweise können der Halteabschnitt und/oder der Dichtungsabschnitt aus einem Metall oder einer Metall-Legierung gebildet und die Feder durch Anspritzen an den Halte- und Dichtungsabschnitt angeformt sein.

Gemäß einer vorteilhaften Weiterbildung der Erfindung (Anspruch 6) hat der Anschluss-Stutzen einen ersten kleindurchmessrigen (Auslauf-) Kanalabschnitt, der stromauf in einen zweiten mitteldurchmessrigen Kanalabschnitt unter Ausbildung der Ventilsitz- / Anlagefläche überspringt, der wiederum stromauf in einen dritten großdurchmessrigen (Einlauf-) Kanalabschnitt übergeht, der den Aufnahmeraum bildet. Der Übergang zwischen mittel- und großdurchmessrigem Kanalabschnitt ist angefast, um eine verklemmfreie Axialbewegung des Ventilkörpers/Dichtelements zu gewährleisten.

Der mitteldurchmessrige Kanalabschnitt (mit Anfassung) sowie die Ventilsitz/Anlagefläche bilden zusammen die Ventilsitzeinrichtung gemäß der Erfindung, wie dies nachfolgend noch näher beschrieben wird.

Vorzugsweise befindet sich das Dichtelement bereits in der vollständig geschlossenen Ventil-Position, wenn es randseitig dichtend mit dem mitteldurchmessrigen Kanalabschnitt in Eingriff kommt. Die Schließbewegung des Dichtelements wird begrenzt durch in Anlage kommen an eine durch die Erweiterung des Strömungskanals gebildete axiale Ringfläche. Da in der vollständig geöffneten Position des Ventils der Dichtungsabschnitt umströmt und somit ein Verkippen des Dichtungsabschnitts trotz der federelastischen Verbindung möglich ist, weist die Ventilsitzeinrichtung die Anfasung / Einlaufschräge auf, die ein schnelles und zuverlässiges Schließen des Ventils gewährleistet. Alternativ zur genannten Ringfläche kann die Erweiterung statt der genannten Kreisringform die Form eines Kegelstumpfes oder einer Kugelkalotte aufweisen.

Gemäß einer vorteilhaften Weiterbildung der vorliegenden Erfindung (Anspruch 7) weist der Fluidanschluss einen Dichtungskörper, vorzugsweise eine Rundschnurdichtung, O-Ring oder ein durch eine Zwei-Komponenten-Spritztechnik hergestellten Dichtungskörper, auf, der sich um eine äußere Mantelfläche des Dichtungselements/Ventilkörpers oder um eine innere Oberfläche des mitteldurchmessrigen Kanalabschnitts erstreckt und seine Dichtfunktion in Zusammenwirkung mit der inneren Oberfläche des mitteldurchmessrigen Kanalabschnitts bzw. der äußeren Oberfläche des Dichtelements entfaltet.

Somit befindet sich der Dichtungskörper in einem zwischen dem Dichtungsabschnitt/Ventilkörper und dem mitteldurchmessrigen Kanalabschnitt gebildeten Spalt, der durch den Dichtungskörper fluiddicht verschließbar ist.

Gemäß einer vorteilhaften Weiterbildung der vorliegenden Erfindung (Anspruch 8) erstreckt sich der Dichtungskörper in einer Umfangsnut, die an der Mantelfläche des Ventilkörpers / Ventiltellers ausgeformt ist.

Die Umfangsnut unterstützt eine Lagefixierung des Dichtungskörpers in der Längsrichtung und erlaubt eine stabile Befestigung des Dichtungskörpers, insbesondere wenn hierfür ein üblicher, kostengünstiger O-Ring mit rundem Querschnitt Verwendung findet.

Gemäß einer vorteilhaften Weiterbildung der vorliegenden Erfindung (Anspruch 9) ist der Halteabschnitt federelastisch in einer Ringnut des Anschluss-Stutzens aufgenommen, beziehungsweise darin eingeclipt.

Dies erlaubt ein einfaches Befestigen des Ventils in den Anschluss-Stutzen, indem das Ventil / Schließeinheit, mit dem Dichtungsabschnitt voraus, in Strömungsrichtung in den Anschluss-Stutzen (großdurchmessrigen Kanalabschnitt) eingeführt und der Halteabschnitt dann in Richtung der Längsachse nach innen gedrückt wird, so dass sich der Halteabschnitt leicht verbiegt und/oder radial zusammengedrückt wird, bis er in die Ringnut einschnappt. Hierbei befindet sich vorzugsweise der Dichtungsabschnitt nicht in Anlage an der Ringfläche des mitteldurchmessrigen Kanalabschnitts, sondern liegt (in Konstruktionslage) nur dichtend am mitteldurchmessrigen Kanalabschnitt randseitig an. Die Feder verbleibt somit unbelastet und erhält daher ihre Form über einen langen Lagerzeitraum. Die Ringnut besitzt vorzugsweise einen halbkreisförmigen oder V-förmigen Querschnitt, so dass die Ringnut im Querschnitt des Fluidanschlusses wie eine Klammer "( )" oder "□ □" oder "< >" aussieht, die die mittlere Öffnung des Halteabschnitts umklammert. Die Tiefe der Nut und die Steifigkeit des Halteabschnitts bzw. die Federkraft der federelastischen Aufnahme bestimmen die zum Einbau des Halteabschnitts auf ihn auszuübende Kraft ebenso wie die auf den Halteabschnitt von dem Fluid übertragbare Kraft in der Längsrichtung.

Gemäß einer vorteilhaften Weiterbildung der vorliegenden Erfindung (Anspruch 10) ist der Dichtungsabschnitt einteilig mit einer Betätigungshilfe / einem Betätigungsstift verbunden, der sich in Strömungsrichtung von dem Dichtungsabschnitt vorzugsweise stoffeinstückig mit diesem in den Strömungskanal erstreckt.

Dieser Betätigungsstift verlagert den Kontaktpunkt zum mechanischen Öffnen des Ventils durch ein entsprechendes, insbesondere behälterseitiges Gegenstück (rohrförmiger Luerkegel) in Richtung kleindurchmessrigen Kanalabschnitt.

Gemäß einer vorteilhaften Weiterbildung der vorliegenden Erfindung (Anspruch 11) ist der Fluidanschluss ein sogenannter Luer-Anschluss, wobei der Anschluss-Stutzen ein Luer-Außengewinde und einen Luer-Innenkonus aufweist.

Die vorliegende Erfindung wird besser verständlich aus der nachfolgenden Beschreibung vorteilhafter Ausführungsformen in Verbindung mit den beigefügten Zeichnungen. In den Zeichnungen ist:
Fig. 1 eine schematische Querschnittsansicht eines Fluidanschlusses gemäß einer ersten Ausführungsform der vorliegenden Erfindung mit geöffnetem Ventil und eingeführtem Luerkegel;
Fig. 2 eine schematische Querschnittsansicht des Fluidanschlusses von Fig. 1 mit geschlossenem Ventil und ohne Luerkegel; und
Fig. 3 eine schematische Seitenansicht des Ventils der Fign. 1 und 2; und
Fig. 4 eine schematische Seitenansicht einer Modifikation des Ventils der Fign. 1 bis 3.

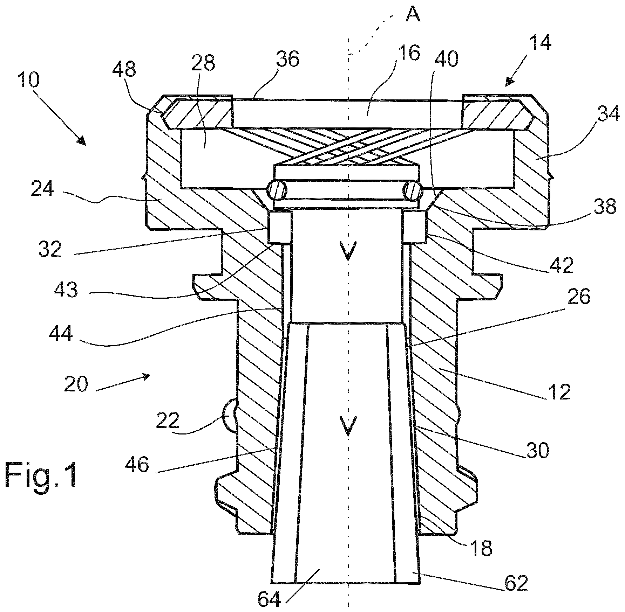

Fig. 1 zeigt einen Fluidanschluss 10 mit einem Anschluss-Stutzen 12 in Form eines Luerlock-Anschluss-Stutzens 12 (Ventilpatrone) und einem darin koaxial zu einer Längsachse A aufgenommenen Ventil 14 (Ventil-Schließeinheit). Der Anschluss-Stutzen 12 ist so ausgelegt, dass er von einem Fluid in der durch Pfeile in Fig. 1 gezeigten Richtung von einer Einlassöffnung 16 zu einer Auslassöffnung 18 durchströmt werden kann.

Der Luerlock-Anschluss-Stutzens 12 besitzt einen Gewindeabschnitt 20 (Auslaufseite) mit einem Außengewinde 22 und einen einstückig mit dem Gewindeabschnitt 20 verbundenen Fluidkammerabschnitt 24 (Einlaufseite). Der Gewindeabschnitt 20 und der Fluidkammerabschnitt 24 begrenzen einen in dem in Fig. 1 dargestellten Querschnitt allgemein T-förmigen Strömungskanal 26, wobei der so genannte Deckstrich des "T" eine Fluidkammer 28 mit großem Querschnitt und der so genannte Stamm des "T" eine Anschluss-Stutzen-Durchführung 30 mit kleinem Querschnitt bildet. Eine innere Umfangswand der Anschluss-Stutzen-Durchführung 30 bildet ferner eine an die Fluidkammer 28 angrenzende Ventilsitzeinrichtung 32 (mittlerer Kanalabschnitt mit mittlerem Querschnitt). Der mittlere Kanalabschnitt 32 erstreckt sich in Richtung der Längsachse A in einem Übergangsbereich zwischen dem Gewindeabschnitt 20 und dem Fluidkammerabschnitt 24.

Die Fluidkammer 28 wird in radialer Richtung von einer hülsenförmigen Umfangswand 34 des Fluidkammerabschnitts 24, stromab beziehungsweise nach unten (die Bezeichnungen "oben", "unten" etc. beziehen sich auf die jeweils beschriebene Figur) von einer kreisringförmigen Stirnfläche des Fluidkammerabschnitts 24 und stromauf beziehungsweise nach oben durch einen kreisringförmigen Halteabschnitt 36 des (eingesetzten) Ventils 14 begrenzt. Der mitteldurchmessrige Kanalabschnitt (Ventilsitzeinrichtung) 32 besitzt einen unten an die Fluidkammer 28 unmittelbar angrenzenden Führungsabschnitt 38 in Form einer Einlaufschräge 40 / Anfasung, welche durch die Mantelfläche eines geraden, sich nach unten verjüngenden Kreiskegelstumpfes ausgebildet ist. Der mitteldurchmessrige Kanalabschnitt 32 besitzt ferner einen unten an den Führungsabschnitt 38 angrenzenden, kreiszylindrischen Abschnitt 42, der den eigentlichen mitteldurchmessrigen Kanalabschnitt der Anschluss-Stutzen-Durchführung 30 bildet. Die Anschluss-Stutzen-Durchführung 30 setzt sich von dem kreiszylindrischen Abschnitt 42 nach unten durch einen letzten kreiszylindrischen Abschnitt 44, dessen Durchmesser kleiner ist als derjenige des kreiszylindrischen Abschnitts 42 ist, und einen konischen Abschnitt 46 mit einem sich nach unten weitenden Durchmesser fort. Zwischen dem mittleren kreiszylindrischen Abschnitt 42 und dem letzten auslaufseitigen kreiszylindrischen Abschnitt 44 ist aufgrund ihrer unterschiedlichen Durchmesser eine radiale Schulter mit einer kreisringförmigen (axial wirkenden) Anlagefläche 43 gebildet, welche den Ventilsitzanschlag der Schließeinheit bildet.

Somit umfasst der Strömungskanal 26 von oben nach unten bzw. in Strömungsrichtung gesehen, die großdurchmessrige Fluidkammer (Aufnahmeraum) 28, den mittleren Kanalabschnitt 32 (= der Führungsabschnitt 28 + der kreiszylindrische Abschnitt 42), den letzten kreiszylindrischen Abschnitt 44 mit kleinem Kanaldurchmesser und den konischen Abschnitt 46.

In der Umfangswand 34 der großdurchmessrigen Fluidkammer 28 ist radial innen eine umlaufende Schnappnut 48 ausgebildet, in der ein korrespondierend ausgebildeter Rand des ringscheibenförmigen Halteabschnitts 36 federelastisch aufgenommen ist. Ein Innendurchmesser des Halteabschnitts 36 definiert dabei einen Durchmesser der Einlassöffnung 16.

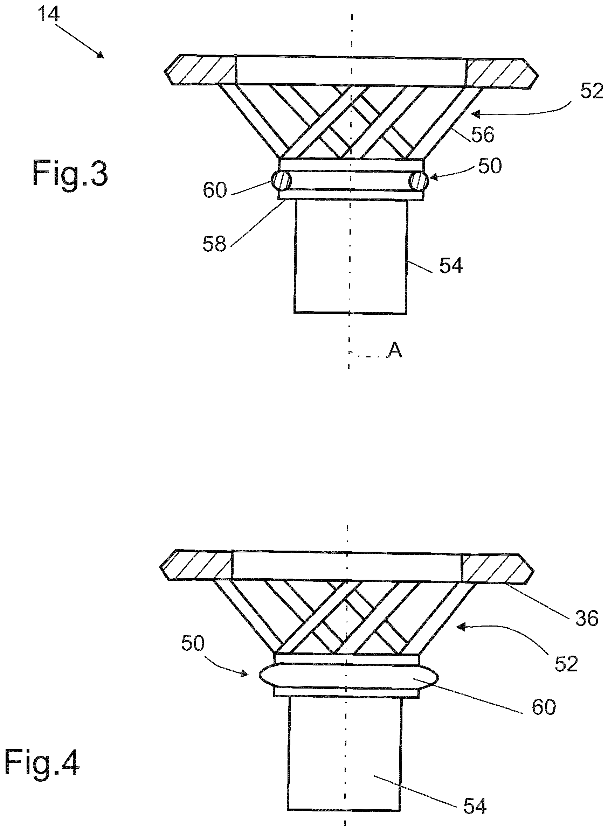

Fig. 3 zeigt eine separate schematische Seitenansicht des Ventils 14 / Schließeinheit der Fig. 1, in der deren Struktur besser erkennbar ist. Das Ventil 14 weist den Halteabschnitt 36 (Federsitzring), einen Dichtungsabschnitt 50 (Ventilkörper / Ventilteller), ein zwischen dem Halteabschnitt 36 und dem Dichtungsabschnitt 50 angeordnetes Federsystem 52 und eine unterhalb des Dichtungsabschnitts 50 angeordnete Betätigungshilfe 54 in Form eines sich nach unten erstreckenden Stifts / Kolbens mit rechteckigem, kreuzförmigen oder ovalem Querschnitt auf. Der Halteabschnitt 36, das Federsystem 52, der Dichtungsabschnitt 50 und die Betätigungshilfe 54 sind einteilig beziehungsweise stoffeinstückig miteinander verbunden und vorzugsweise durch ein spritztechnisches Verfahren zusammen ausgebildet. Das Federsystem 52 weist eine Mehrzahl von äquidistant zueinander um die Längsachse A angeordnete Biegestabfederelemente 56 auf, deren untere Enden entlang eines Umfangsabschnitts des Dichtungsabschnitts 50 mit dem Dichtungsabschnitt (Ventilkörper) 50 verbunden sind, und deren obere Enden mit dem ringförmigen Halteabschnitt 36 verbunden sind.

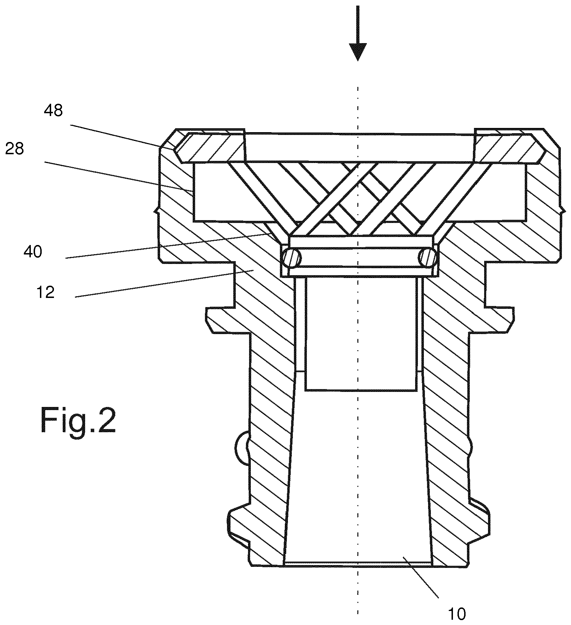

Es ist zu beachten, dass das Federsystem 52 in Fig. 3 in seinem vollständig entspannten Zustand, in Fig. 1 in seinem vollständig angespannten Zustand (vollständig geöffneter Zustand des Ventils 14) und in Fig. 2 in seiner (entspannten) Konstruktionslage (bereits vollständig geschlossener Zustand des Ventils 14) gezeigt ist.

Der Dichtungsabschnitt 50 besitzt eine umlaufende Ringnut 58, in der ein Dichtungskörper 60 in Form eines O-Rings eingepasst ist.

Alternativ, wie es in Fig. 4 gezeigt ist, kann auf die umlaufende Ringnut 58 verzichtet werden, so dass der Dichtungskörper 60 auf einer kreiszylindrischen, äußeren Umfangsoberfläche des Dichtungsabschnitts (Ventilkörper) 50 aufliegt und gegebenenfalls darauf angeklebt ist.

Um das Ventil 14 in dem Anschluss-Stutzen 12 zu befestigen, wird das Ventil (Schließeinheit) 14 mit dem Dichtungsabschnitt 50 voraus in die Fluidkammer 28 eingeführt und durch zentrisches Eindrücken entlang der Längsachse A des Halteabschnitts 36 der Dichtungsabschnitt 50 in der Ventilsitzeinrichtung 32 (lose) 43 positioniert, bis der Halteabschnitt 36 in die Schnappnut 48 einschnappt oder einrastet.

Nachfolgend ist die Funktion des Fluidanschlusses 10 beschrieben.

Im unbetätigten Zustand des Fluidanschlusses 10 (/Konstruktionslagen) d. h. im geschlossenen Zustand des Ventils 14 liegt der Dichtungsabschnitt 50 durch Federsystem 52 in nicht vorgespannter Anlage mit dem kreiszylindrischen Abschnitt 42, jedoch nicht an der kreisringförmigen Anlagefläche 43 des kreiszylindrischen Abschnitts 42 an, wobei der Dichtungskörper 60 fluiddicht den kleindurchmessrigen Strömungskanal 26 verschließt (vgl. Fig. 2). Die Fluidkammer 28 ist mit einem Fluid gefüllt, das im Betrieb zusätzlich auf den Ventilkörper 50 drückt und diesen somit weiter ggf. gegen den Ventilsitzanschlag 43 verschiebt.

Indem ein rohrförmiger Luerkegel 62 entgegen der Strömungsrichtung, also von unten nach oben in Fig. 1, in die Anschluss-Stutzen-Durchführung 30 eingeführt und durch ihn eine nach oben gerichtete Kraft auf die Betätigungshilfe 54 und damit den Dichtungsabschnitt 50 ausgeübt wird, bewegt sich der Dichtungsabschnitt 50 wahlweise gegen die Federkraft des Federsystems 52 und den hydrostatischen Druck des Fluids nach oben, wodurch der Strömungskanal 26 geöffnet wird. Das Fluid kann in diesem Zustand durch die Einlassöffnung 16 hindurch, den Dichtungsabschnitt 50 umströmend, in die Anschluss-Stutzen-Durchführung 30 gelangen und durch eine Durchführung 64 in dem (hohlen) Luerkegel 62 weitergeleitet werden.

Wird der Luerkegel 62 entfernt / zurückgezogen sorgen der Strömungsdruck des Fluids zusammen mit der Federkraft des Federsystems 52 für eine schnelle Abwärts- und damit Schließbewegung des Ventilkörpers 50, die von dem Führungsabschnitt 38 klemmfrei geführt wird. Eine beginnende Abdichtung wird teilweise schon erreicht, wenn der Dichtring 50 auf dem angefasten Bereich 40 des Führungsabschnitts 32 aufsitzt. Das wäre somit eine mögliche Vorstufe zur kompletten Abdichtung, die dann wirken könnte, wenn bereits Flüssigkeit aus dem Behälter entnommen wurde und der Druck auf das Ventil damit nicht mehr so stark ist.

Wie es oben vorstehend bereits erwähnt ist, ist jede Bewegung des Dichtungsabschnitts 50 gegenüber dem Halteabschnitt 36 entlang der Längsachse A von einer Drehbewegung des Dichtungsabschnitts 50 um diese Achse begleitet, wodurch die Schließ- und Öffnungsbewegung des Dichtungsabschnitts 50 erleichtert ist.

Aufgrund der vorspannfreien Positionierung des Ventilkörpers 50 im kreiszylindrischen Abschnitt 42 der Ventilsitzeinrichtung 32 kann die Dichtwirkung über einen langen Lagerzeitraum erhalten werden, ohne eine plastische Deformation des Federsystems zu erwarten. Daher kann das Federsystem stoffeinstückig mit dem Ventilkörper und dem Federsitz beispielsweise aus einem Kunststoffmaterial angefertigt sein.

### Bezugszeichenliste

- 10: Fluidanschluss
- 12: Luerlock-Anschluss-Stutzen
- 14: Ventil / Schließeinheit
- 16: Einlassöffnung
- 18: Auslassöffnung
- 20: Gewindeabschnitt
- 22: Außengewinde von 20
- 24: Fluidkammerabschnitt
- 26: Strömungskanal
- 28: Fluidkammer / großdurchmessriger Kanalabschnitt
- 30: Anschluss-Stutzen-Durchführung / kleindurchmessriger Kanalabschnitt
- 32: Ventilsitzabschnitt / mitteldurchmessriger Kanalabschnitt
- 34: Umfangswand
- 36: Halteabschnitt / Federsitz
- 38: Führungsabschnitt
- 40: Einlaufschräge / Anfasung
- 42: kreiszylindrischer Abschnitt
- 44: letzter kreiszylindrischer Abschnitt
- 46: konischer Abschnitt
- 48: Schnappnut
- 50: Dichtungsabschnitt / Ventilkörper / Ventilteller
- 52: Federsystem
- 54: Betätigungshilfe
- 56: Biegefederstabelement
- 58: Ringnut
- 60: Dichtungskörper
- 62: Luerkegel
- 64: Durchführung in 62

## Patentansprüche

1. Fluidanschluss mit einem Anschluss-Stutzen (12), in dessen innerem Strömungs-Längskanal (26) eine axial wirkende Ventilsitzeinrichtung (32) ausgeformt ist, und einer Ventil-Schließeinheit (14) bestehend aus einem in den Strömungs-Längskanal (26) fest, vorzugsweise durch Verclippen eingesetzten Federsitz-Halteelement (36), einer Federanordnung (52) und einem mit der Ventilsitzeinrichtung (32) zusammenwirkenden, von der Federanordnung (52) gehaltenen Ventilkörper (50), die stoffeinstückig miteinander ausgebildet sind, wobei die Ventilsitzeinrichtung (32) eine axial wirkende Ventilsitz-Ringfläche (43) hat, die durch eine zu der Längsachse konzentrische, kreiszylindrische Erweiterung (42) des Strömungskanals gebildet ist, **dadurch gekennzeichnet, dass** der Fluidanschluss einen Dichtungskörper (60), der sich in Umfangsrichtung entlang einer radial äußeren Oberfläche des Ventilkörpers (50) oder einer inneren Oberfläche der kreiszylindrischen Erweitung erstreckt und seine Dichtfunktion in Zusammenwirkung mit der inneren Oberfläche der kreiszylindrischen Erweiterung oder einer äußeren Oberfläche des Ventilkörpers (50) in Konstruktionslage bei nicht vorgespannter Federanordnung (52) entfaltet.

2. Fluidanschluss nach Anspruch 1, **dadurch gekennzeichnet, dass** die Federanordnung (52) durch wenigstens eine Feder, insbesondere wenigstens eine Blattfeder, insbesondere wenigstens eine geschwungene Blattfeder oder eine V-förmige Blattfeder oder eine Z-förmige Blattfeder oder eine ellipsenförmige Blattfeder, gebildet wird.

3. Fluidanschluss nach Anspruch 1, **dadurch gekennzeichnet, dass** die Federanordnung (52) durch wenigstens eine Feder, insbesondere eine schräg gestellte Biegestabfeder gebildet wird.

4. Fluidanschluss nach Anspruch 2 oder 3, **dadurch gekennzeichnet, dass** das Federsitz-Halteelement (36) im Wesentlichen in Form eines Rings und der Ventilkörper (50) im Wesentlichen in Form einer Scheibe oder Tellers ausgebildet ist, und die federelastische Wirkung durch eine Mehrzahl von Federn erzeugt wird, die im Wesentlichen äquidistant entlang des Federsitz-Halteelements (36) und eines Umfangsabschnitts des Ventilkörpers (50) geschaltet sind.

5. Fluidanschluss nach Anspruch 2 oder 3, **dadurch gekennzeichnet, dass** die wenigstens eine Feder durch Urformen, insbesondere Spritzgießen, gebildet und mit dem Federsitz-Halteelement (36) und dem Ventilkörper (50) einstückig verbunden ist.

6. Fluidanschluss nach Anspruch 1, **dadurch gekennzeichnet, dass** sich an die zu der Längsachse konzentrische, kreiszylindrische Erweiterung (42) des Strömungskanals in Richtung hin zum Fluidanschluss-Einlauf eine radial sich aufweitende Einlaufschräge (38) zur Führung des Ventilkörpers (50) beim Schließvorgang anschließt.

7. Fluidanschluss nach Anspruch 6, **dadurch gekennzeichnet, dass** der Dichtungskörper (60), eine Rundschnurdichtung, O-Ring oder ein durch eine Zwei-Komponenten-Spritztechnik hergestelltes Dichtelement aufweist.

8. Fluidanschluss nach Anspruch 1 oder 7, **dadurch gekennzeichnet, dass** der Dichtskörper (60) in eine Umfangsnut (58) eingesetzt ist, die im Ventilkörper (50) ausgeformt ist.

9. Fluidanschluss nach Anspruch 1, **dadurch gekennzeichnet, dass** das Federsitz-Halteelement (36) federelastisch in einer Ring- / Schnappnut (48) des Anschluss-Stutzens (12) aufgenommen ist.

10. Fluidanschluss nach Anspruch 1, **dadurch gekennzeichnet, dass** der Ventilkörper (50) einteilig mit einer Betätigungshilfe (54) verbunden ist, die sich in Strömungsrichtung von dem Ventilkörper 50 stromab in den Strömungskanal (26) erstreckt.

11. Fluidanschluss nach einem der Ansprüche 1 bis 10, **dadurch gekennzeichnet, dass** der Fluidanschluss ein Luerlock-Anschluss ist, wobei der Anschluss-Stutzen ein Luer-Außengewinde (22) und einen Luer-Innenkonus (46) aufweist.

## Claims

1. Fluid connection with a connection socket (12), inside the inner flow-longitudinal channel (26) of which an axially acting valve seat unit (32) is formed, and a valve-closure unit (14) consisting of a spring seat-retaining element (36) fixedly inserted in the flow-longitudinal channel (26), preferably by clipping, a spring assembly (52) and a valve body (50) interacting with the valve seat unit (32) and held by the spring assembly (52), the spring assembly and valve body being integrally formed with each other, wherein the valve seat unit (32) has an axially acting valve seat ring face (43) which is formed by a circular cylindrical extension (42) of the flow channel concentric to the longitudinal axis, **characterised in that** the fluid connection comprises a sealing body (60) extending in the circumferential direction along a radial outer surface of the valve body (50) or an inner surface of the circular cylindrical extension and unfolding its sealing function in conjunction with the inner surface of the circular cylindrical extension or an outer surface of the valve body (50) in construction state when the spring assembly (52) is not preloaded.

2. Fluid connection according to claim 1, **characterised in that** the spring assembly (52) is formed by at least one spring, in particular at least one leaf spring, in particular at least one curved leaf spring or a V-shaped leaf spring or a Z-shaped leaf spring or an elliptical leaf spring.

3. Fluid connection according to claim 1, **characterised in that** the spring assembly (52) is formed by at least one spring, in particular a flexible rod spring in an inclined position.

4. Fluid connection according to claim 2 or 3, **characterised in that** the spring seat-retaining element (36) has substantially the shape of a ring and the valve body (50) has substantially the shape of a disc or plate, and the spring elastic effect is generated by a plurality of springs switched substantially equidistantly along the spring seat-retaining element (36) and a circumferential section of the valve body (50).

5. Fluid connection according to claim 2 or 3, **characterised in that** the at least one spring is formed by casting, in particular injection-moulding, and is integrally connected to the spring seat-retaining element (36) and the valve body (50).

6. Fluid connection according to claim 1, **characterised in that** a radially widening inlet taper (38) joins the circular cylindrical extension (42) of the flow channel concentric to the longitudinal axis in the direction towards the fluid connection inlet for guiding the valve body (50) during the closing process.

7. Fluid connection according to claim 6, **characterised in that** the sealing body (60) comprises a round cord sealing, 0-ring or a sealing element manufactured using a two-component-injection technique.

8. Fluid connection according to claim 1 or 7, **characterised in that** the sealing body (60) is inserted into a circumferential groove (58) formed in the valve body (50).

9. Fluid connection according to claim 1, **characterised in that** the spring seat-retaining element (36) is spring elastically received in a circular/snap groove (48) of the connection socket (12).

10. Fluid connection according to claim 1, **characterised in that** the valve body (50) is integrally connected to an actuation aid (54) extending in the flow direction from the valve body (50) downstream into the flow channel (26).

11. Fluid connection according to any of claims 1 to 10, **characterised in that** the fluid connection is a Luerlock connection, wherein the connection socket comprises a Luer outer thread (22) and a Luer internal cone (46).

## Revendications

1. Raccord de fluide comprenant un manchon de raccordement (12), dans le canal longitudinal d'écoulement interne (26) duquel est formé un dispositif de siège de soupape (32) opérant axialement et une unité de fermeture de soupape (14) constituée d'un élément de support de siège de ressort (36) inséré de manière fixe dans le canal longitudinal d'écoulement (26), de préférence par encliquetage, un agencement de ressort(s) (52) et un corps de soupape (50) coopérant avec le dispositif de siège de soupape (32) et supporté par l'agencement de ressort(s) (52), qui sont formés conjointement d'un seul tenant de matériau, dans lequel le dispositif de siège de soupape (32) a une surface annulaire de siège de soupape (43) opérant axialement, qui est formé par un évasement cylindrique circulaire (42) du canal d'écoulement concentrique avec l'axe longitudinal, **caractérisé en ce que** le raccord de fluide déploie un corps d'étanchéité (60) qui s'étend dans la direction périphérique le long d'une surface radialement externe du corps de soupape (50) ou d'une surface interne de l'évasement cylindrique circulaire et sa fonction d'étanchéité en coopération avec la surface interne de l'évasement cylindrique circulaire ou avec une surface externe du corps de soupape (50) en position de construction lorsque l'agencement de ressort(s) (52) n'est pas précontraint.

2. Raccord de fluide selon la revendication 1, **caractérisé en ce que** l'agencement de ressort(a) (52) est formé par au moins un ressort, en particulier au moins un ressort à lames, en particulier au moins un ressort à lames incurvé ou un ressort à lames en forme de V ou un ressort à lames en forme de Z ou un ressort à lames en forme d'ellipse.

3. Raccord de fluide selon la revendication 1, **caractérisé en ce que** l'agencement de ressort(s) (52) est formé par au moins un ressort, en particulier un ressort à boudin en position oblique.

4. Raccord de fluide selon la revendication 2 ou 3, **caractérisé en ce que** l'élément de support de siège de ressort (36) se présente sensiblement sous la forme d'une bague et le corps de soupape (50) sensiblement sous la forme d'un disque ou d'un plateau et l'action élastique du ressort est produite par une pluralité de ressorts qui sont installés sensiblement à équidistance le long de l'élément de support de siège de ressort (36) et d'une section périphérique du corps de soupape (50).

5. Raccord de fluide selon la revendication 2 ou 3, **caractérisé en ce que** le au moins un ressort est formé par formage primitif, en particulier par moulage par injection, et est relié d'un seul tenant avec l'élément de support de siège de ressort (36) et le corps de soupape (50).

6. Raccord de fluide selon la revendication 1, **caractérisé en ce qu'**un chanfrein d'entrée (38) s'évasant radialement se raccorde à l'évasement cylindrique annulaire (42) du canal d'écoulement concentrique avec l'axe longitudinal dans la direction de l'entrée du raccord de fluide pour guider le corps de soupape (50) lors de l'opération de fermeture.

7. Raccord de fluide selon la revendication 6, **caractérisé en ce que** le corps d'étanchéité (60) présente un joint torique 0-ring ou un élément d'étanchéité fabriqué par une technique d'injection à deux composants.

8. Raccord de fluide selon la revendication 1 ou 7, **caractérisé en ce que** le corps d'étanchéité (60) est inséré dans une rainure périphérique (58) qui est formée dans le corps de soupape (50).

9. Raccord de fluide selon la revendication 1, **caractérisé en ce que** l'élément de support de siège de ressort (36) est reçu de manière élastique dans une rainure d'encliquetage annulaire (48) du manchon de raccordement (12).

10. Raccord de fluide selon la revendication 1, **caractérisé en ce que** le corps de soupape (50) est relié d'un seul tenant avec un élément auxiliaire de commande (54) qui s'étend dans la direction d'écoulement du corps de soupape (50) en aval dans le canal d'écoulement (26).

11. Raccord de fluide selon l'une quelconque des revendications 1 à 10, **caractérisé en ce que** le raccord de fluide est un raccord de verrouillage Luer, dans lequel le manchon de raccordement présente un filet externe Luer (22) et un cône interne Luer (46).
